# FASCICULE DE BREVET EUROPEEN

(11) **EP 2 533 774 B1**
(45) Date de publication et mention de la délivrance du brevet: **31.01.2018**
(21) Numéro de dépôt: 11708533.2
(22) Date de dépôt: 10.02.2011
(51) Int. Cl.: A61K 31/165, A61P 25/22

(54) **UTILISATION DE L'AGOMELATINE POUR L'OBTENTION DE MEDICAMENTS DESTINES AU TRAITEMENT DU TROUBLE OBSESSIONNEL COMPULSIF (TOC)**
VERWENDUNG VON AGOMELATIN ZUR HERSTELLUNG VON MEDIKAMENTEN ZUR BEHANDLUNG OBSESSIVER ZWANGSSTÖRUNGEN
USE OF AGOMELATINE FOR THE PREPARATION OF DRUGS FOR TREATING OBSESSIVE COMPULSIVE DISORDER (OCD)

(30) Priorité: 11.02.2010 FR 1000560
(43) Date de publication de la demande: 19.12.2012
(73) Titulaire: Les Laboratoires Servier, 92284 Suresnes Cedex (FR)
(72) Inventeur: LAIGLE, Laurence, F-78600 Maisons Laffitte (FR); MOCAER, Elisabeth, F-92200 Neuilly sur Seine (FR); MILLAN, Mark, J., F-78230 Le Pecq (FR)
(86) Numéro de dépôt international: PCT/FR2011/000080
(87) Numéro de publication internationale: WO 2011/098689

(56) Documents cités:
- EP-A1- 1 564 202
- EP-A1- 1 842 535
- WO-A1-2005/002562
- WO-A1-2007/137227
- WO-A2-2006/096434
- WO-A2-2008/035177
- STEIN ET AL: "P.4.a.016 Agomelatine in generalized anxiety disorder: a randomized, placebo-controlled, study", EUROPEAN NEUROPSYCHOPHARMACOLOGY, ELSEVIER SCIENCE PUBLISHERS BV, AMSTERDAM, NL LNKD- DOI:10.1016/S0924-977X(07)70784-8, vol. 17, 1 octobre 2007 (2007-10-01), pages S509-S510, XP022335436, ISSN: 0924-977X
- LOISEAU F ET AL: "Effects of melatonin and agomelatine in anxiety-related procedures in rats: Interaction with diazepam", EUROPEAN NEUROPSYCHOPHARMACOLOGY, ELSEVIER SCIENCE PUBLISHERS BV, AMSTERDAM, NL LNKD- DOI:10.1016/J.EURONEURO.2005.11.007, vol. 16, no. 6, 1 août 2006 (2006-08-01), pages 417-428, XP025110895, ISSN: 0924-977X [extrait le 2006-08-01]
- LANDEROS-WEISENBERGER A ET AL: "Dimensional predictors of response to SRI pharmacotherapy in obsessive-compulsive disorder", JOURNAL OF AFFECTIVE DISORDERS, ELSEVIER BIOCHEMICAL PRESS, AMSTERDAM, NL, vol. 121, no. 1-2, 1 février 2010 (2010-02-01), pages 175-179, XP026816585, ISSN: 0165-0327 [extrait le 2009-07-03]
- MOCAËR ELISABETH ET AL: "Development of a new antidepressant : agomelatine", M/S MEDECINE SCIENCES, SOCIETE DES PERIODIQUES FLAMMARION, PARIS, FR, vol. 21, no. 10, 1 octobre 2005 (2005-10-01), pages 888-893, XP009074822, ISSN: 0767-0974

## Description

La présente invention concerne l'utilisation de l'agomélatine ou *N*-[2-(7-méthoxy-1-naphtyl)éthyl]acétamide de formule (I) : 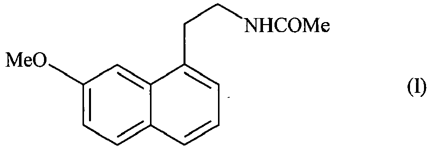 ainsi que ses hydrates, formes cristallines et sels d'addition à un acide ou à une base pharmaceutiquement acceptable, pour l'obtention de médicaments destinés au traitement du Trouble Obsessionnel Compulsif (TOC).

L'agomélatine ou *N*-[2-(7-méthoxy-1-naphtyl)éthyl]acétamide présente la double particularité d'être d'une part agoniste sur les récepteurs du système mélatoninergique et d'autre part antagoniste du récepteur 5-HT_{2C}. Ces propriétés lui confèrent une activité dans le système nerveux central et plus particulièrement dans le traitement de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité.

L'agomélatine, sa préparation et son utilisation en thérapeutique ont été décrits dans les brevets européens EP 0 447 285 et EP 1 564 202.

La demanderesse a présentement découvert que l'agomélatine ou *N*-[2-(7-méthoxy-1-naphtyl)éthyl]acétamide, ainsi que ses hydrates, formes cristallines et sels d'addition à un acide ou à une base pharmaceutiquement acceptable, possédait des propriétés intéressantes permettant de l'utiliser dans le traitement du Trouble Obsessionnel Compulsif (TOC).

Le Trouble Obsessionnel Compulsif (TOC) est une pathologie se définissant par l'existence d'obsessions ou de compulsions. Cette pathologie répond à des critères parfaitement définis et constitue une entité nosographique à part entière (300-3, Trouble-DSM IV - Manuel Diagnostique et Statistique des Troubles Mentaux, 4ème édition, American Psychiatric Association).

Les Obsessions sont définies par des pensées, impulsions ou représentations mentales récurrentes qui, à certains moments, sont ressenties comme intrusives et inappropriées et qui entraînent une détresse importantes. Ce ne sont pas seulement des préoccupations excessives concernant les problèmes de la vie réelle. Le patient d'une part fait des efforts pour les ignorer ou les réprimer, et d'autre part reconnaît qu'elles proviennent de sa propre activité mentale et qu'elles sont excessives ou irraisonnées.

Les Compulsions sont des comportements répétitifs, ou des actes mentaux destinés à neutraliser ou diminuer le sentiment de détresse ou à empêcher un événement ou une situation redoutés. Les Compulsions sont soit sans relation réaliste avec ce qu'elles se proposent de neutraliser ou prévenir soit manifestement excessives.

Les Obsessions ou Compulsions sont à l'origine de sentiments marqués de détresse, d'une perte de temps considérable. Elles interfèrent notablement avec le fonctionnement socioprofessionnel et les activités habituelles du patient.

Le Trouble Obsessionnel Compulsif est une pathologie chronique, qui ne résulte pas des effets physiologiques directs d'une substance. Sa prévalence vie entière est de l'ordre de 2 à 3%. (Kaplan A. et al., Psychiatric Services, 2003, 54 (8)).

Il n'existe pas actuellement de traitement réellement satisfaisant du Trouble Obsessionnel Compulsif. Le plus souvent les patients sont traités par l'antidépresseur clomipramine, antidépresseur tricyclique, ou principalement par des inhibiteurs de la recapture de la sérotonine (SSRI) en association avec une thérapie cognitive et comportementale. Cependant, les traitements SSRI induisent des effets secondaires notables comme des troubles gastro-intestinaux tels que nausées, anorexie, perte de poids, une dysfonction sexuelle ou un syndrome sérotoninergique. D'autre part, leur efficacité n'est pas immédiate mais apparaît dans un délai de 15 jours à 3 semaines de traitement, et seulement 20 % des patients répondent à ces traitements.

Il subsiste donc toujours un réel besoin pour de nouveaux traitements permettant d'améliorer la vie de patients souffrant de Trouble Obsessionnels Compulsifs (TOC).

La demanderesse a présentement découvert que l'agomélatine, de par ses caractéristiques pharmacologiques et notamment une excellente tolérance observée dans les études cliniques conduites chez près de 3900 patients, pouvait être utilisée dans le traitement du Trouble Obsessionnel Compulsif (TOC).
Notamment l'agomélatine est dépourvu des effets secondaires associés aux psychotropes classiques. Parmi ces effets, le syndrome de discontinuation observé à l'arrêt du traitement avec les psychotropes classiques est inexistant avec l'agomélatine, ce qui en fait un traitement de choix dans cette indication.

L'invention concerne donc l'utilisation de l'agomélatine, ainsi que ses hydrates, formes cristallines et sels d'addition à un acide ou à une base pharmaceutiquement acceptable, pour l'obtention de compositions pharmaceutiques destinées au traitement du Trouble Obsessionnel Compulsif (TOC).

Particulièrement, l'invention concerne l'utilisation de l'agomélatine obtenue sous la forme cristalline II décrite dans la demande de brevet EP 1 564 202, pour l'obtention de compositions pharmaceutiques destinées au traitement du Trouble Obsessionnel Compulsif (TOC).

Les compositions pharmaceutiques seront présentées sous des formes convenant aux administrations par voie orale, parentérale, transcutanée, nasale, rectale, perlinguale, et notamment sous forme de préparations injectables, comprimés, comprimés sublinguaux, glossettes, gélules, capsules, tablettes, suppositoires, crèmes, pommades, gels dermiques, etc...

Outre l'agomélatine, les compositions pharmaceutiques selon l'invention contiennent un ou plusieurs excipients ou véhicules choisis parmi des diluants, des lubrifiants, des liants, des agents de désintégration, des absorbants, des colorants, des édulcorants, etc...

A titre d'exemple et de manière non limitative, on peut citer :
◆ *pour les diluants :* le lactose, le dextrose, le sucrose, le mannitol, le sorbitol, la cellulose, la glycérine,
◆ *pour les lubrifiants* : la silice, le talc, l'acide stéarique et ses sels de magnésium et de calcium, le polyéthylène glycol,
◆ *pour les liants* : le silicate d'aluminium et de magnésium, l'amidon, la gélatine, la tragacanthe, la méthylcellulose, la carboxyméthylcellulose de sodium et la polyvinylpyrrolidone,
◆ *pour les désintégrants* : l'agar, l'acide alginique et son sel de sodium, les mélanges effervescents.

La posologie utile varie selon le sexe, l'âge et le poids du patient, la voie d'administration, la nature de l'affection et des traitements éventuellement associés et s'échelonne entre 1 mg et 50 mg d'agomélatine par 24 heures.

De manière préférentielle, la dose journalière d'agomélatine sera de 25 mg par jour, avec possibilité d'augmenter à 50 mg par jour.

### Composition pharmaceutique :

Formule de préparation pour 1000 comprimés dosés à 25 mg :

| | |
|---|---|
| N-[2-(7-méthoxy-1-naphtyl)éthyl]acétamide | 25 g |
| Lactose monohydrate | 62 g |
| Stéarate de Magnésium | 1,3 g |
| Povidone | 9 g |
| Silice colloïdale anhydre | 0,3 g |
| Cellulose sodium glycolate | 30 g |
| Acide stéarique | 2,6 g |

### Etude préclinique

Des études pré-cliniques ont été conduites avec un modèle de Trouble Obsessionnel Compulsif (TOC) qui a confirmé le potentiel de l'agomélatine dans le traitement de cette pathologie. L'enfouissement spontané des billes chez la souris (« Marble Burying ») est un comportement répétitif considéré comme très pertinent pour la maladie du TOC, et son inhibition suggère une activité thérapeutique dans son traitement (Witkin J.M., Current Protocols Neurosciences, 2008, Chapter 9, Unit 9.30). L'agomélatine aux doses de 10, 40 et 80 mg/kg après administration intraperitonéale a fortement et de façon dose-dépendente diminué l'enfouissement spontané des billes chez la souris indiquant un potentiel thérapeutique dans le traitement des TOCs. L'étude s'est effectuée comme ci-après. Des souris mâles de souche NMRI (Iffa-Credo, L'Arbresle, France), pesant 20-25 g le jour de l'expérience, ont étés placées individuellement dans des boîtes en Macrolon (30 x 18 x 19 cm) contenant 5 cm de sciure et recouvertes par une plaque en plexiglass perforée. Vingt-quatre billes en verre " oeil de chat" ont été réparties régulièrement sur la sciure à la périphérie de la boîte. Au terme de 30 minutes d'exploration libre, les animaux ont étés retirés de la boîte et le nombre de billes enfouies comptabilisé. L'agomélatine ou le véhicule (contrôle) ont été injectés 30 minutes avant le début du test.

Les résultats obtenus, donnés en nombre de billes enfouies, sont les suivants :
Véhicule : 20,2 ± 0,6 (n = 14)
Agomelatine 10 mg/kg : 19,2 ± 1,3 (n = 6)
Agomelatine 40 mg/kg : 15,3 ± 3,0 (n = 6)
Agomelatine 80 mg/kg: 4,6 ± 1,9 (n = 5)
Analyse de variance: F (3,33) = 23,4 P < 0,01. Aux doses de 40 et 80 mg/kg d'agomelatine, P < 0,05 *vs* vehicule (test du Dunnett).

Les résultats obtenus montrent une activité statistiquement significative de l'agomélatine sur un modèle représentatif du Trouble Obsessionnel Compulsif.

### Etude clinique

Une étude clinique comparant l'agomélatine au placebo est réalisée chez 80 patients ambulatoires de plus de 18 ans et de moins de 65 ans, présentant un diagnostic primaire de Trouble Obsessionnel Compulsif selon les critères du DSM IV TR. Les patients doivent présenter à l'entrée dans l'étude un score de 20 ou plus sur l'échelle Y-BOCS (Yale Brown Obsessive Compulsive Scale) et avoir été précédemment traités pour leur trouble Obsessionnel Compulsif par un inhibiteur de recapture de la sérotonine (IRS). Les patients doivent présenter un score de sévérité de la dépression inférieur à 24 sur l'échelle de dépression MADRS.
Il s'agit d'une étude en double aveugle controlée versus placebo pour une durée de traitement de 16 semaines. Les patients sont randomisés soit dans le bras placebo soit dans le bras agomelatine 25 mg avec possibilité d'augmentation de dose d'agomelatine à 50 mg en cas de non réponse après 8 semaines de traitement (critère de non-réponse: diminution du score total de la Y-BOCS de moins de 20%).
Le critère principal de jugement de l'efficacité est la réduction du score total à l'échelle Y-BOCS. Les autres critères de jugement évaluant la sévérité de l'état obsessionel et/ou compulsif sont les scores à la NIMH-OC (National Institute of Mental Health Obsessive-Compulsive scale) et à la CGI-S (Clinical Global Impression-Severity) ainsi que l'amélioration de cet état par la CGI-I (Clinical Global Impression-Improvement). La présence de symptômes dépressifs et leur évolution sont analysées par l'échelle de dépression MADRS à l'initiation du traitement et après 16 semaines de traitement.
La réponse est définie comme une baisse de 35% du score total à l'échelle Y-BOCS ainsi qu'un score de 1 ou 2 à la CGI-I. La rémission est définie par un score à l'échelle Y-BOCS inférieur ou égal à 10 et à la CGI-S inférieur ou égal à 2.

Les résultats observés confirment l'efficacité de l'agomélatine dans le traitement du Trouble Obsessionnel Compulsif (TOC) ainsi que son bon profil d'acceptabilité.

## Revendications

1. Utilisation de l'agomélatine ou *N*-[2-(7-méthoxy-1-naphtyl)éthyl]acétamide ou un de ses hydrates, formes cristallines et sels d'addition à un acide ou à une base pharmaceutiquement acceptable, comme seul principe actif, pour l'obtention d'un médicament destiné au traitement du Trouble Obsessionnel Compulsif (TOC).

2. Utilisation selon la revendication 1 **caractérisée en ce que** l'agomélatine est obtenue sous la forme cristalline II telle que décrite dans la demande de brevet EP1564202.

3. Compositions pharmaceutiques consistant en de l'agomélatine ou un de ses hydrates, formes cristallines et sels d'addition à un acide ou à une base pharmaceutiquement acceptable, seule ou en combinaison avec un ou plusieurs excipients pharmaceutiquement acceptables, destiné à être utilisé dans le traitement du Trouble Obsessionnel Compulsif (TOC).

4. Composition pharmaceutique destinée a être utilisé selon la revendication 3 **caractérisée en ce que** l'agomélatine est obtenue sous la forme cristalline II telle que décrite dans la demande de brevet EP1564202.

5. Agomélatine ou N-[2-(7-méthoxy-l-naphtyl)éthyl]acétamide ou un de ses hydrates, formes cristallines ainsi que ses sels d'addition à un acide ou à une base pharmaceutiquement acceptable destiné à être utilisé dans le traitement du Trouble Obsessionnel Compulsif (TOC).

6. Forme cristalline II de l'agomélatine telle que décrite dans la demande de brevet EP1564202 destinée à être utilisée dans le traitement du Trouble Obsessionnel Compulsif (TOC).

## Patentansprüche

1. Verwendung von Agomelatin oder *N*-[2-(7-Methoxy-1-naphthyl)-ethyl]-acetamid oder eines seiner Hydrate, Kristallformen und Additionssalze mit einer pharmazeutisch annehmbaren Säure oder Base als alleiniger Wirkstoff zur Herstellung eines Arzneimittels, das bestimmt ist zu Behandlung von Zwangsstörung .

2. Verwendung nach Anspruch 1, **dadurch gekennzeichnet, dass** Agomelatin in der Kristallform II erhalten wird, wie es in der Patentanmeldung EP 1 564 202 beschrieben ist.

3. Pharmazeutische Zubereitungen, bestehend aus Agomelatin oder eines seiner Hydrate, Kristallformen und Additionssalze mit einer pharmazeutisch annehmbaren Säure oder Base allein oder in Kombination mit einem oder mehreren pharmazeutisch annehmbaren Hilfsstoffen zur Verwendung bei der Behandlung von Zwangsstörung .

4. Pharmazeutische Zubereitung zur Verwendung gemäß Anspruch 3, **dadurch gekennzeichnet, dass** Agomelatin in der Kristallform II erhalten wird, wie es in der Patentanmeldung EP 1 564 202 beschrieben ist.

5. Agomelatin oder *N*-[2-(7-Methoxy-1-naphthyl)-ethyl]-acetamid oder eines seiner Hydrate, Kristallformen sowie Additionssalze mit einer pharmazeutisch annehmbaren Säure oder Base zur Verwendung bei der Behandlung von Zwangsstörung .

6. Kristallform II von Agomelatin, wie es in der Patentanmeldung EP 1 564 202 beschrieben ist, zur Verwendung bei der Behandlung von Zwangsstörung .

## Claims

1. Use of agomelatine or *N*-[2-(7-methoxy-1-naphthyl)ethyl]acetamide or one of its hydrates, crystalline forms and addition salts with a pharmaceutically acceptable acid or base, as the only active ingredient, for obtaining a medicament for the treatment of obsessive compulsive disorder (OCD).

2. Use according to claim 1, **characterised in that** agomelatine is obtained in crystalline form II as described in patent application EP1564202.

3. Pharmaceutical compositions consisting of agomelatine or one of its hydrates, crystalline forms and addition salts with a pharmaceutically acceptable acid or base, on its own or in combination with one or more pharmaceutically acceptable excipients, for use in the treatment of obsessive compulsive disorder (OCD).

4. Pharmaceutical composition for use according to claim 3, **characterised in that** agomelatine is obtained in crystalline form II as described in patent application EP1564202.

5. Agomelatine or N-[2-(7-methoxy-1-naphthyl)ethyl]acetamide or one of its hydrates, crystalline forms as well as its addition salts with a pharmaceutically acceptable acid or base for use in the treatment of obsessive compulsive disorder (OCD).

6. Crystalline form II of agomelatine as described in patent application EP1564202 for use in the treatment of obsessive compulsive disorder (OCD).
